(19) Europäisches Patentamt
European Patent Office
Office européen des brevets

(11) Veröffentlichungsnummer: **0 030 701**
**B1**

(12) # EUROPÄISCHE PATENTSCHRIFT

(45) Veröffentlichungstag der Patentschrift: **20.04.83**

(51) Int. Cl.[3]: **C 07 C 39/14**, C 07 C 37/14, C 07 C 43/23, C 07 C 39/38

(21) Anmeldenummer: **80107743.9**

(22) Anmeldetag: **09.12.80**

(54) **Verfahren zur Herstellung von 1-(Aralkyl)-2-naphtholen.**

(30) Priorität: **14.12.79 DE 2950290**

(43) Veröffentlichungstag der Anmeldung: **24.06.81 Patentblatt 81/25**

(45) Bekanntmachung des Hinweises auf die Patenterteilung: **20.04.83 Patentblatt 83/16**

(84) Benannte Vertragsstaaten: **BE CH DE FR GB LI NL**

(56) Entgegenhaltungen:
**DE A 1 518 460**
**GB A 1 257 005**

(73) Patentinhaber: **BASF Aktiengesellschaft**
**Carl-Bosch-Strasse 38**
**D-6700 Ludwigshafen (DE)**

(72) Erfinder: **Merger, Franz, Dr.**
**Max-Slevogt-Strasse 25**
**D-6710 Frankenthal (DE)**
Erfinder: **Nestler, Gerhard, Dr.**
**Van-Leyden-Strasse 17**
**D-6700 Ludwigshafen (DE)**

**EP 0 030 701 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 1-(Aralkyl)-2-naphtholen

Die Erfindung betrifft ein Verfahren zur Herstellung von 1-(Aralkyl)-2-naphtholen durch Umsetzung von 2-Naphtholen mit Styrolen in Gegenwart einer Carbonsäure.

1-alkylierte und 1-benzylierte 2-Naphthole werden bis jetzt hauptsächlich durch Umsetzung von Alkalinaphtholaten mit Alkoholen bei hoher Temperature oder durch mehrstufige Synthesen in mäßiger Ausbeute gewonnen (Chemistry Letters 1975, Seiten 1 019 bis 1 020).

In US-PS 2 714 120 wird die Bildung von 1-(Aralkyl)-2-naphtholen durch Umsetzung von 2-Naphtholen mit Styrolen in Gegenwart von verdünnter Schwefelsäure oder Sulfonsäuren, insbesondere p-Toluolsulfonsäure, in einer Ausbeute von 16,5 Prozent beschrieben.

Es ist bekannt, daß in der Regel die Alkylierung von 2-Naphthol mit Olefinen oder Alkoholen in Gegenwart von anorganischen Säuren oder organischen Sulfonsäuren vor allem zu 6-alkylierten 2-Naphtholen führt. In J. Org. Chem., Band 17 (1952), Seiten 243 bis 248 wird die Herstellung von 6-α-Phenyläthyl)-2-naphthol durch Umsetzung von 2-Naphthol mit Styrol in Gegenwart von Schwefelsäure in einer Ausbeute von 92 Prozent beschrieben. J. Org. Chem., Band 16, Seiten 185 bis 191 (1951) zeigt die Bildung von 6-Cyclohexyl-2-naphthol durch Umsetzung von 2-Naphthol mit Cyclohexynol in Gegenwart von Phosphorsäure.

Die GB-PS 1 257 005 beschreibt die Umsetzung von aromatischen Hydroxyverbindungen, z. B. Naphthole, mit Olefinen, z. B. Styrol, bei 100 bis 300 °C in Gegenwart von polymerem Aluminium Alkoholat als Katalysator. Weitere Katalysatoren sind Kondensationsprodukte von Aluminiumhydroxid und Aluminiumalk-, -cycloalk- oder -aryloxiden und weiterhin mit vorgenannten Aluminiumalkoholaten Aluminiumoxid.

Nachteilig ist, daß eine besondere Katalysatorherstellung notwendig ist und die Katalysatoren die Wirtschaftlichkeit des Verfahrens verschlechtern. Auch zeigen die Beispiele, daß längere Zeit bei Temperaturen von über 200 °C, z. B. 225 bis 250 °C, umgesetzt werden muß.

Es wurde nun gefunden, daß man 1-(Aralkyl)-2-naphthole der Formel

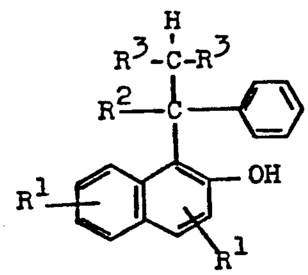

(I)

worin die einzelnen Reste $R^1$, $R^2$ und $R^3$ gleich oder verschieden sein können und jeweils ein Wasserstoffatom oder einen aliphatischen Rest bedeuten, $R^1$ auch eine Alkoxygruppe oder ein Halogenatom bezeichnet, durch Umsetzung von 2-Naphtholen der Formel

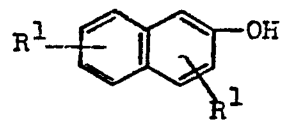

(II)

worin $R^1$ die vorgenannte Bedeutung besitzt, mit Styrolen der Formel

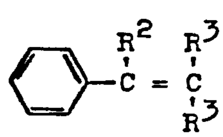

(III)

worin $R^2$ und $R^3$ die vorgenannte Bedeutung besitzen in Gegenwart einer Säure vorteilhaft erhält, wenn man in Gegenwart einer Carbonsäure umsetzt.

Die Umsetzung kann für den Fall der Verwendung von Styrol und 2-Naphthol durch die folgenden Formeln wiedergegeben werden :

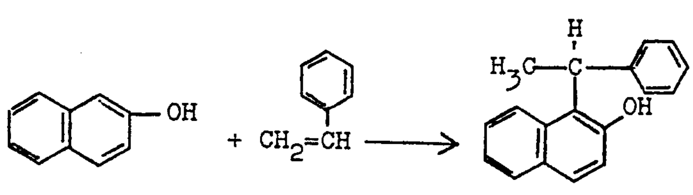

Im Vergleich zu den bekannten Verfahren liefert das Verfahren nach der Erfindung überraschend auf einfacherem und wirtschaftlicherem Wege 1-(Aralkyl)-2-naphthole in besserer Raum-Zeit-Ausbeute und Reinheit. Das Reaktionsgemisch kann vorteilhaft ohne Abtrennung des Katalysators direkt destillativ aufgearbeitet werden.

Die Ausgangsstoffe werden in stöchiometrischem Verhältnis oder im Überschuß, vorzugsweise in einem Verhältnis von 0,5 bis 2, vorteilhaft von 0,8 bis 1,2 Mol Ausgangsstoff III je Mol Naphthol II, umgesetzt. Bevorzugte Ausgangsstoffe II, III und dementsprechend bevorzugte Endstoffe I sind solche, in deren Formeln die einzelnen Reste $R^1$, $R^2$ und $R^3$ gleich oder verschieden sein können und jeweils ein Wasserstoffatom, einen Alkylrest mit 1 bis 8, insbesondere 1 bis 4 Kohlenstoffatomen bedeuten, $R^1$ auch eine Alkoxygruppe mit 1 bis 8, insbesondere 1 bis 4 Kohlenstoffatomen, ein Chloratom oder ein Bromatom bezeichnet. Die genannten Reste können noch durch unter den Reaktionsbedingungen inerte Gruppen, z. B. Alkylgruppen oder Alkoxygruppen mit 1 bis 4 Kohlenstoffatomen, substituiert sein.

Folgende Naphthole kommen z. B. als Ausgangsstoffe II in Betracht : Chlor-, Brom-, Methyl-, Äthyl-, n-Propyl-, Isopropyl-, n-Butyl-, Isobutyl-, sek.-Butyl-β-naphthol, wobei der Halogen- oder Alkylsubstituent in 3-, 4-, 5-, 6-, 7- oder 8-Stellung sitzt ; entsprechende Methyl-, Äthyl-, n-Propyl-, n-Butyl-, Isobutyl-äther der in vorgenannten Stellungen eine weitere Hydroxylgruppe tragenden β-Naphthole ; in 3,4-, 4,5-, 4,6-, 6,7-Stellung durch Chloratome, Bromatome, Methyl-, Äthyl-, n-Propyl-, Isopropyl-, n-Butyl-, Isobutyl-, sek.-Butyl-gruppen disubstituiertes β-Naphthol ; entsprechende β-Naphthole mit 2 vorgenannten, aber unterschiedlichen Resten, z. B. 4-Äthyl-6-chlor-2-naphthol, 3-Methyl-4-methoxy-2-naphthol ; vorzugsweise β-Naphthol.

Es können z. B. folgende Styrole als Ausgangsstoffe III verwendet werden : Styrol ; in α- oder β-Stellung einfach oder in α, β- oder in β, β-Stellung gleich oder unterschiedlich zweifach oder in α, β, β-Stellung gleich oder unterschiedlich dreifach durch die Methyl-, Äthyl-, Propyl-, Isopropyl-, Butyl-gruppe substituierte Styrole.

Die Umsetzung wird im allgemeinen bei einer Temperatur von 100 bis 180 °C, vorzugsweise von 120 bis 150 °C, mit Unterdruck, Überdruck oder drucklos, diskontinuierlich oder kontinuierlich durchgeführt. Zweckmäßig verwendet man keine zusätzlichen Lösungsmittel ; gegebenenfalls kommen aber auch, z. B. zur Erniedrigung der Viskosität des Reaktionsgemisches, unter den Reaktionsbedingungen inerte Lösungsmittel in Betracht. Als Lösungsmittel kommen z. B. in Frage : Halogenkohlenwasserstoffe, insbesondere Chlorkohlenwasserstoffe, z. B. Tetrachloräthylen, 1,1,2,2-Tetrachloräthan oder 1,1,1,2-Tetrachloräthan, Dichlorpropan, Methylenchlorid, Dichlorbutan, Chloroform, Tetrachlorkohlenstoff, Tetrachloräthan, 1,1,1-Trichloräthan oder 1,1,2-Trichloräthan, Trichloräthylen, Pentachloräthan, 1,2-Dichloräthan, 1,1-Dichloräthan ; Tetrahydrofuran, Dioxan ; aromatische Kohlenwasserstoffe, z. B. Toluol, Äthylbenzol. o-, m-, p-Xylol, Isopropylbenzol, Chlorbenzol, Methylnaphthalin ; und entsprechende Gemische. Zweckmäßig verwendet man das Lösungsmittel in einer Menge von 10 bis 1 000 Gewichtsprozent, vorzugsweise von 50 bis 100 Gewichtsprozent, bezogen auf Olefin III.

**Als Carbonsäuren kommen araliphatische, aromatische und vorzugsweise aliphatische Carbonsäuren, sowohl polybasische, zweckmäßig dibasische, als auch vorteilhaft monobasische Carbonsäuren in** Betracht. Vorteilhaft sind Carbonsäuren mit einem Dissoziationsexponenten $pK_S$ kleiner als 5, vorzugsweise 0,1 bis 4, insbesondere 0,1 bis 2. Man verwendet vorzugsweise solche der Formel

$$R^4-\overset{O}{\overset{\|}{C}}-OH \qquad (III)$$

worin $R^4$ ein Wasserstoffatom, einen gegebenenfalls durch Chloratome, Fluoratome, eine Cyanogruppe, eine Nitrogruppe und/oder eine weitere Carboxylgruppe substituierten Alkylrest mit 1 bis 8, insbesondere 1 bis 4 Kohlenstoffatomen, einen Alkylphenylrest mit jeweils 7 bis 12 Kohlenstoffatomen oder einen Phenylrest bedeutet. Die vorgenannten Reste können noch durch unter den Reaktionsbedingungen inerte Gruppen und/oder Atome, z. B. Bromatome, Chloratome, Alkylgruppen mit 1 bis 4 Kohlenstoffatomen, Cyangruppen, Carboxylgruppen, Carbalkoxygruppen, Acetylgruppen, Nitrogruppen, substituiert sein. Die Carbonsäure wird vorzugsweise in einem Verhältnis von 0,01 bis 1, insbesondere 0,05 bis 0,5 Äquivalenten Carbonsäure je Mol Ausgangsstoff II verwendet.

Beispielsweise sind folgende Carbonsäuren geeignet : aliphatische Carbonsäuren wie Chloressigsäure, Dichloressigsäure, Trichloressigsäure, Oxalsäure, Ameisensäure, Cyanessigsäure, Essigsäure, Trifluoressigsäure, Monobromessigsäure, α-Chlorpropionsäure, Malonsäure, α, α-Dichlorpropionsäure, Malonsäure ; aromatische Carbonsäuren wie Benzoesäure, 2,3-, 2,4-, 2,5-, 2,6-Dichlorbenzoesäure, o-, m- bzw. p-Chlorbenzoesäure, Phthalsäure, o-, m- bzw. p-Nitrobenzoesäure. Bevorzugt verwendet man Dichloressigsäure, Trichloressigsäure, Trifluoressigsäure oder Oxalsäure.

Die Reaktion kann wie folgt durchgeführt werden : Ein Gemisch von Ausgangsstoff II und III, Carbonsäure und gegebenenfalls Lösungsmittel wird während 2 bis 5 Stunden bei der Reaktionstemperatur gehalten. Dann wird aus dem Reaktionsgemisch der Endstoff in üblicher Weise, z. B. durch Destillation, isoliert.

Die nach dem Verfahren der Erfindung herstellbaren 1-(Aralkyl)-2-naphthole I sind wertvolle

Ausgangsstoffe für die Herstellung von Farbstoffen, Schädlingsbekämpfungsmitteln, Pharmazeutika, Salben, Emulgatoren, Dispergiermitteln, Stabilisatoren, hydraulischen Flüssigkeiten, Weichmachern, Korrosionsinhibitoren, Desinfektionsmitteln, Pflanzenschutzmitteln, Riechstoffen. 1-alkylierte 2-Naphthole finden als Kupplungskomponenten in der Farbfotographie (DE-AS 10 99 350) Verwendung. Bezüglich der Verwendung wird auf die vorgenannten Veröffentlichungen verwiesen.

Die in den folgenden Beispielen aufgeführten Teile bedeuten Gewichtsteile.

Beispiel 1

In einem Rührreaktor wird ein Gemisch aus 144 Teilen 2-Naphthol und 5 Teilen Oxalsäure unter Rühren auf 130 °C erhitzt und 104 Teile Styrol innerhalb einer Stunde kontinuierlich zugeführt. Anschließend wird drei Stunden bei 130 °C gerührt. Nach Beendigung der Umsetzung wird das Gemisch im Vakuum destilliert. Man erhält 213 Teile (86 % der Theorie) 1-(α-Phenyläthyl)-2-naphthol (Kp 172 bis 176 °C/0,2 mbar). Der Umsatz beträgt 90 Prozent.

Vergleichsbeispiel 2

In einem Rührreaktor wird ein Gemisch aus 144 Teilen 2-Naphthol und 1 Teil Schwefelsäure (96-gewichtsprozentig) unter Rühren auf 120 °C erhitzt und innerhalb von zwei Stunden 104 Teile Styrol kontinuierlich zugeführt. Anschließend wird das Gemisch zwei Stunden bei 120 °C gerührt. Entsprechend der gaschromatographischen Analyse besteht das Reaktionsgemisch aus :

12 Gewichtsprozent 2-Naphthol
42 Gewichtsprozent 6- und 3-(α-Phenyläthyl)-2-naphthol (Mengenverhältnis 1 : 1)
6 Gewichtsprozent Dinaphthyläther

Es verbleiben : di- und höheralkylierte Naphthole.

Vergleichsbeispiel 3

In einem Rührreaktor wird ein Gemisch aus 144 Teilen 2-Naphthol und 4 Teilen Phosphorsäure (85-gewichtsprozentig) unter Rühren auf 120 °C erhitzt und innerhalb von zwei Stunden 104 Teile Styrol kontinuierlich zugeführt. Anschließend wird das Gemisch zwei Stunden bei 120 °C gerührt. Entsprechend der gaschromatographischen Analyse besteht das Reaktionsgemisch aus :

6 Gewichtsprozent 2-Naphthol
37 Gewichtsprozent 1-(α-Phenyläthyl)-2-naphthol
46 Gewichtsprozent 6- und 3-(α-Phenyläthyl)-2-naphthol (Mengenverhältnis 1 : 1)

Es verbleiben : di- und höheralkylierte Naphthole.

**Anspruch**

Verfahren zur Herstellung von 1-(Aralkyl)-2-naphtholen der Formel

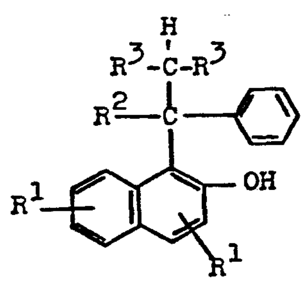

(I)

worin die einzelnen Reste $R^1$, $R^2$ und $R^3$ gleich oder verschieden sein können und jeweils ein Wasserstoffatom oder einen aliphatischen Rest bedeuten, $R^1$ auch eine Alkoxygruppe oder ein Halogenatom bezeichnet, durch Umsetzung von 2-Naphtholen der Formel

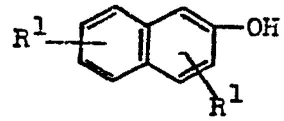

(II)

worin $R^1$ die vorgenannte Bedeutung besitzt, mit Styrolen der Formel

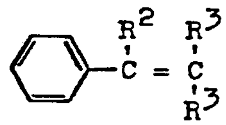

(III)

worin $R^2$ und $R^3$ die vorgenannte Bedeutung besitzen, in Gegenwart einer Säure, dadurch gekennzeichnet, daß man in Gegenwart einer Carbonsäure umsetzt.

**Claim**

A process for the preparation of 1-(aralkyl)-2-naphthols of the formula

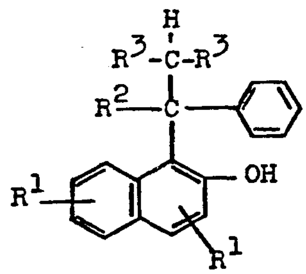

(I)

where the individual radicals $R^1$, $R^2$ and $R^3$ may be identical or different and each is hydrogen or an aliphatic radical, and $R^1$ may also be alkoxy or halogen, by reacting a 2-naphthol of the formula

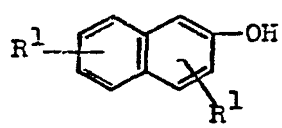

(II)

where $R^1$ has the above meanings, in the presence of an acid, with a styrene of the formula

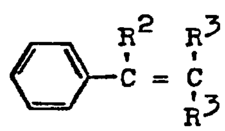

(III)

where $R^2$ and $R^3$ have the above meanings, wherein the reaction is carried out in the presence of a carboxylic acid.

**Revendication**

Procédé de préparation de 1-(aralcoyl) 2-naphtols de la formule

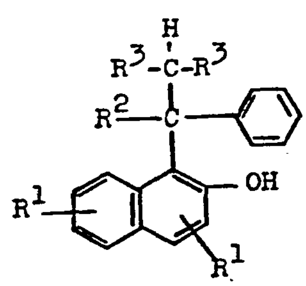

(I)

dans laquelle les restes $R^1$, $R^2$ et $R^3$, qui peuvent être identiques ou différents, désignent chacun un atome d'hydrogène ou un groupe aliphatique, $R^1$ pouvant de plus désigner un atome d'halogène ou un

radical alcoxy, par réaction d'un 2-naphtol de la formule

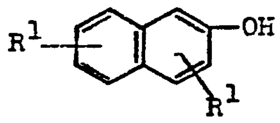

(II)

dans laquelle $R^1$ possède la signification définie, et d'un styrène de la formule

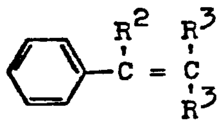

(III)

dans laquelle $R^2$ et $R^3$ possèdent la signification définie, en présence d'un acide, caractérisé en ce que la réaction est effectuée en présence d'un acide carboxylique.